# DEMANDE DE BREVET EUROPEEN

(11) **EP 0 628 289 A1**
(43) Date de publication de la demande: **14.12.1994**
(21) Numéro de dépôt: 94420156.5
(22) Date de dépôt: 31.05.1994
(51) Int. Cl.: A61B 17/60

(54) **Fixateur externe modulaire évolutif pour immobilisation d'un foyer de fracture**

(30) Priorité: 01.06.1993 FR 9306796
(71) Demandeur: Hardy, Jean-Marie, F-19600 Larches (FR)
(72) Inventeur: Hardy, Jean-Marie, F-19600 Larches (FR)
(74) Mandataire: Thivillier, Patrick

(57) **Abrégé**

Le fixateur comprend :
- des mâchoires standards (3) conformées pour être fixées coaxialement selon différentes positions angulaires avec un corps (1,20) et/ou d'autres mâchoires,
- des mâchoires intermédiaires (7) conformées pour être fixées coaxialement selon différentes positions angulaires avec d'autres mâchoires ou une mâchoire et un corps,
- des mâchoires (8) conformées pour être fixées avec le corps et/ou d'autres mâchoires dans un plan orthogonal,
- des mâchoires (9) dont une partie est orientable pour le positionnement angulaire des broches d'ancrage,
- un corps d'allongement (20) apte à être monté entre deux mâchoires,
- un module d'alignement (23).

## Description

On connaît différents types de fixateur externes pour interventions orthopédiques, afin de procéder à des ostéosynthèses d'un membre quelconque. Ces fixateurs ont pour but d'assurer la réduction et la contention d'une fracture.

Généralement, un fixateur comprend des mâchoires montées en combinaison avec un corps. Chaque mâchoire recoit des broches d'ancrage pour être fixée de part et d'autre du foyer de fracture. La plupart des fixateurs connus ne sont pas modulaires, de sorte qu'il n'est pas possible de les adapter à l'évolution des cas pathologiques à traiter. Il en résulte une application figée lors de la première intervention. De même, il n'est pas possible d'adapter le fixateur au fur et à mesure du traitement et de l'évolution de la fracture considérée.

Le fixateur décrit dans la demande de brevet FR 2 683 446, dont le demandeur de la présente est également titulaire, remédie à ces inconvénients et permet d'assurer l'immobilisation d'une fracture avec conservation des mouvements d'articulation, ainsi que l'immobilisation en position. Pour l'essentiel, cette demande de brevet décrit un fixateur externe entièrement modulable, et qui comprend des éléments faisant office de mâchoires recevant les broches d'ancrage, pour être fixées dans une position déterminée, de part et d'autre du foyer de fracture. Les mâchoires sont aptes à recevoir, après fixation, au moins un corps avec capacité d'orientation angulaire contrôlée et de blocage dans cette position, au moyen de tiges d'accouplement.

Les tiges d'accouplement entre le corps et les mâchoires, sont constituées par une portée cylindrique faisant office de piston et destinée à être engagée avec capacité de coulissement et de blocage en translation dans un agencement complémentaire du corps. L'une des extrémités de cette portée présente une tête sphérique apte à coopérer dans un logement complémentaire formé dans les mâchoires avec capacité de blocage angulaire.

A partir de cette conception de base, il est apparu nécessaire d'étendre et d'améliorer les caractéristiques définies. Notamment, il a été conçu et mis au point, selon l'invention, un fixateur externe modulaire, évolutif et simple à utiliser.

La modularité permet de proposer un montage adapté à chaque cas clinique à traiter, tandis que son caractère évolutif permet de modifier facilement et à volonté, la configuration du fixateur pendant l'évolution du cas clinique traité. Il est également possible, si nécessaire, d'assurer la mobilisation contrôlée avec ou sans distraction.

Enfin, le fixateur selon l'invention peut être posé sur fracture réduite ou non, sa mise en place étant particulièrement simple.

Pour résoudre ces différents problèmes et atteindre de tels objectifs, il a été conçu et mis au point un fixateur externe qui comprend :
- des mâchoires standards conformées pour être fixées coaxialement selon différentes positions angulaires avec le corps et/ou d'autres mâchoires
- des mâchoires intermédiaires conformées pour être fixées coaxialement selon différentes positions angulaires avec d'autres mâchoires ou une mâchoire et un corps
- des mâchoires conformées pour être fixées avec le corps et/ou d'autres mâchoires dans un plan orthogonal
- des mâchoires dont une partie est orientable pour le positionnement angulaire des broches d'ancrage
- un corps d'allongement apte à être monté entre deux mâchoires
- un module d'alignement

Pour résoudre le problème posé d'assurer le blocage en translation du corps par rapport aux tiges, et pour résoudre le problème posé d'assurer le blocage dans une position angulaire déterminée des mâchoires par rapport au corps, les tiges des pistons sont de différentes longueurs et présentent une portée méplate coopérant avec une vis de blocage du corps, les extrémités débordantes desdites tiges qui présentent la tête sphérique, coopérant avec des empreintes en creux complémentaires des mâchoires, sont bloquées en position angulaire par des moyens du type clavette.

Plus particulièrement, le problème posé d'assurer l'accouplement des mâchoires en bout des tiges, avec la possibilité de les orienter après accouplement dans la position angulaire désirée et de les bloquer dans cette position, est résolu en ce que les moyens de blocage des têtes sphériques sont constitués par deux douilles engagées chacune dans un trou débouchant dans l'empreinte recevant la tête sphérique en y étant indexée angulairement, lesdites douilles étant assujetties à une vis pour être rapprochées coaxialement en vue d'exercer une force de pression sur ladite tête, en combinaison avec des empreintes profilées formées aux extrémités desdites douilles.

Pour résoudre le problème posé d'orienter à 90° une mâchoire par rapport à une mâchoire standard notamment, les mâchoires, conformées pour être fixées avec le corps et/ou d'autres mâchoires, dans un plan orthogonal, présentent au moins une empreinte formée dans l'épaisseur de l'une de ses faces latérales, pour l'engagement et la fixation de la tête sphérique d'une tige piston de liaison.

Pour résoudre le problème posé d'augmenter la stabilité des montages il est apparu nécessaire, dans certains cas, de pouvoir disposer les broches d'ancrage d'une manière divergente. Pour résoudre un tel problème, la mâchoire orientable présente une partie fixe pour l'engagement de broches d'ancrage et une partie mobile avec capacité de blocage en position angulaire, ladite partie mobile recevant également des broches d'ancrage.

Avantageusement, la partie mobile présente, à une de ses extrémités, un axe de pivotement engagé dans un chambrage interne du corps de la mâchoire et, à l'autre extrémité, une portée tronconique creuse engagée dans un chambrage formé dans un élément rapporté du corps, un moyen de blocage coopérant avec l'alésage de la portée tronconique.

Pour résoudre le problème posé d'obtenir un allongement progressif entre deux mâchoires, le fixateur comprend un corps d'allongement qui présente, à l'une de ses extrémités au moins, un trou taraudé recevant la tige d'un piston qui est filetée pour être déplacée en translation par un écrou bloqué en translation au niveau de ladite extrémité.

Pour résoudre le problème posé de faciliter la réduction d'une fracture, le module d'alignement comprend deux parties oblongues accouplées entre elles avec capacité de désaxation latérale l'une des parties recevant la tige d'un piston coopérant avec un corps, l'autre partie recevant une tête sphérique coopérant avec une mâchoire.

Pour résoudre le problème posé de fixer à volonté sur le corps et/ou les mâchoires, des barres de rigidification, le corps et/ou les mâchoires présentent une empreinte oblongue destinée à recevoir une portée complémentaire d'un élément support présentant des agencements de fixation au niveau de ladite empreinte, ledit élément support étant apte à recevoir les parties d'un collier de serrage recevant des barres de rigidification entre plusieurs corps et/ou mâchoires.

L'invention est exposée, ci-après plus en détail à l'aide des dessins annexés, dans lesquels:

La figure 1 est une vue en perspective d'un corps monté entre deux mâchoires

La figure 2 est une vue montrant les tiges pistons d'accouplement entre un corps et les mâchoires.

La figure 3 est une vue en perspective d'une mâchoire standard.

La figure 4 est une vue en perspective d'une mâchoire intermédiaire.

La figure 5 est une vue en perspective d'une mâchoire à 90°.

La figure 6 est une vue montrant un exemple de montage du fixateur avec une mâchoire à 90°.

La figure 7 est une vue en perspective d'une mâchoire à divergence.

La figure 8 est une vue en coupe longitudinale d'une mâchoire à divergence.

La figure 9 est une vue en perspective de la mâchoire à divergence, équipée des broches d'ancrage.

La figure 10 est une vue postérieure correspondant à la figure 9.

La figure 11 est une vue en perspective montrant l'accouplement d'une tête sphérique dans l'empreinte complémentaire d'une mâchoire.

La figure 11a est une vue montrant les éléments de blocage de la tête sphérique.

La figure 12 est une vue montrant le principe d'accouplement d'une tête sphérique.

La figure 13 est une vue en perspective d'un exemple de montage, utilisant des barres de mobilisation.

La figure 14 est une vue de face montrant différentes tailles des éléments support.

La figure 15 est une vue montrant les principaux éléments constitutifs des éléments support.

La figure 16 est une vue en perspective montrant le montage d'un élément support sur un corps.

La figure 17 est une vue en perspective montrant le montage d'une barre de rigidification sur un élément support.

La figure 18 est une vue en perspective montrant le montage d'une barre de rigidification entre deux mâchoires.

La figure 19 est une vue en perspective montrant l'accouplement de deux mâchoires au moyen d'un corps d'allongement.

La figure 20 est une vue en perspective du module d'alignement, considéré en vue de dessus.

La figure 21 est une vue correspondant à la figure 20 considéré en vue de dessous.

La figure 22 est une vue en perspective montrant un montage du fixateur, avec le module d'alignement.

Les figures 23, 24 et 25 sont des vues respectivement de profil, de face et en plan, d'un montage diaphysaire mono-corps au niveau tibial.

Les figures 26, 27 et 28 sont des vues respectivement de profil, de face et en plan, d'un montage diaphysaire bi-barres au niveau tibial.

Les figures 29, 30 et 31 sont des vues respectivement de profil, de face et en plan, d'un montage épiphysaire supérieur au niveau tibial.

Les figures 32, 33, 34, et 35 sont des vues de différents montages diaphysaires, respectivement considérées de face, de profil, avec barre de rigidification et relais sur le corps et sans relais sur le corps.

Les figures 36, 37 et 38 sont des vues de montages épiphysaires au moyen de broches divergentes et d'un corps d'allongement.

Les figures 39 et 40 sont des vues montrant l'adaptation du fixateur au niveau de la courbure du fémur.

Les figures 41, 42 et 43 sont des vues respectivement en plan, de face et de profil, d'un montage du fixateur au niveau du bassin.

Les figures 44 et 45 sont des vues en perspective montrant l'immobilisation de l'articulation coxo fémorale.

Les figures 46, 47 et 48 sont des vues en perspective montrant un ligamentotaxis du genou.

Les figures 49, 50 et 51 sont des vues en perspective montrant un ligamentotaxis de la cheville.

Les figures 52, 53, 54, 55, 56, 57, 58, 59, 60, 61 et 62 sont des vues en perspective montrant la réduction d'une fracture, au moyen du module d'alignement.

Les figures 63, 64, 65, 66, 67, 68 et 69 sont des vues en perspective montrant la réduction d'une fracture à dominante de translation au moyen du module d'alignement.

Les figures 70, 71, 72 et 73 sont des vues en perspective montrant l'allongement diaphysaire fémoral au moyen du corps d'allongement.

On rappelle, pour une meilleure compréhension de la suite de la description, que le fixateur comprend un corps (1) apte à recevoir des tiges d'accouplement (2) pour le montage de mâchoires (3). Les mâchoires (3) reçoivent des broches d'ancrage (4) pour la fixation de l'ensemble ainsi constitué de part et d'autre du foyer de fracture. D'une manière parfaitement connue, chaque mâchoire présente un corps (3a) recevant une partie rapportée (3b) agencée en combinaison avec la partie correspondante du corps, pour l'engagement des broches d'ancrage. Des vis (5) ou autres, assurent la solidarisation de l'ensemble.

Le corps (1) se présente sous forme d'un bloc parallélépipèdique présentant au moins un alésage pour le montage, à libre coulissement, des tiges d'accouplement (2). Les tiges sont de différentes longueurs et de sections cylindriques, et présentent une portée méplate (2a), coopérant avec une vis de blocage (6) engagée dans le corps. L'extrémité des tiges (2) présente une tête sphérique (2b) apte à coopérer dans un logement complémentaire, formé dans les mâchoires avec capacité de blocage angulaire.

A partir de cette conception de base, le fixateur peut utiliser, en fonction du cas pathologique à traiter, des mâchoires standards (3), des mâchoires intermédiaires (7), des mâchoires dites à 90° (8) et des mâchoires divergentes (9).

Les mâchoires standards (3) présentent, à partir de l'une de leurs faces transversales, une empreinte (3a) pour l'engagement et l'articulation des têtes sphériques (2a) des tiges pistons (2). Ce type de mâchoires est parfaitement connu et ressort de l'enseignement de la demande de brevet précitée FR 2 683 446.

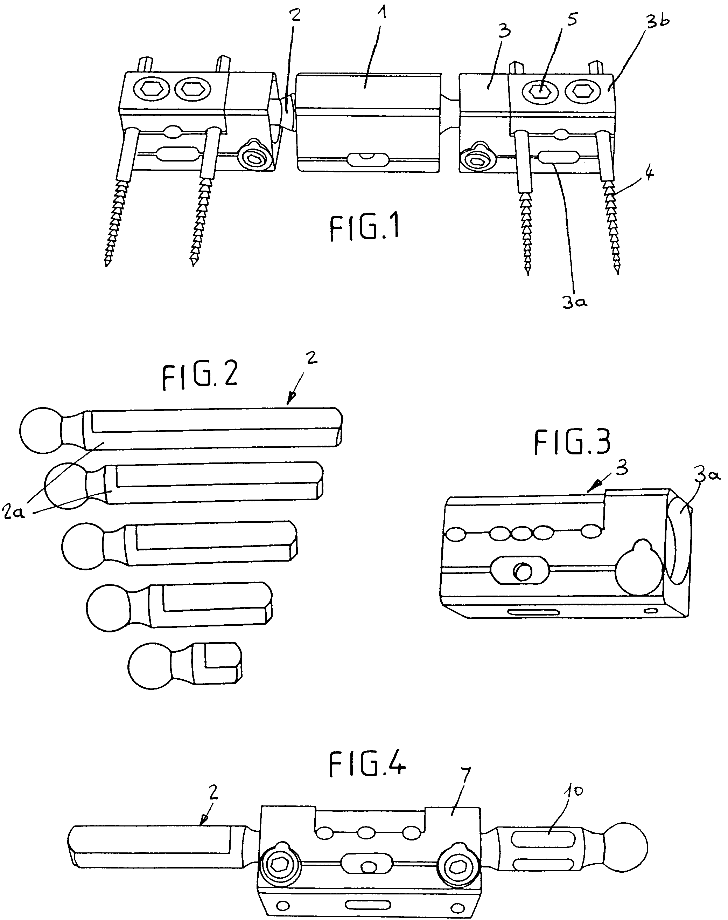

Les mâchoires intermédiaires (7), de la même façon que les mâchoires standards (3), présentent, à chacune de leur extrémité, un logement pour le montage des têtes sphériques. Dans ce but, une mâchoire intermédiaire est montée entre deux éléments auxquels elle est reliée, soit par une tige piston (2), lorsqu'elle est accouplée à un corps, soit par une barre de mobilisation (10), lorsqu'elle est accouplée vers une autre mâchoire (figure 4).

Les mâchoires dites à 90° (8) sont conformées pour être fixées avec le corps (1) et/ou les mâchoires (3) ou (7), dans un plan orthogonal. Dans ce but, chaque mâchoire (8) présente au moins une empreinte (8a) formée dans l'épaisseur de l'une de ses faces latérales. Cette empreinte (8a) est conformée pour recevoir la tête sphérique d'une tige d'accouplement (2). Cette mâchoire (8) permet donc une liaison à 90° par rapport au piston qu'elle reçoit.

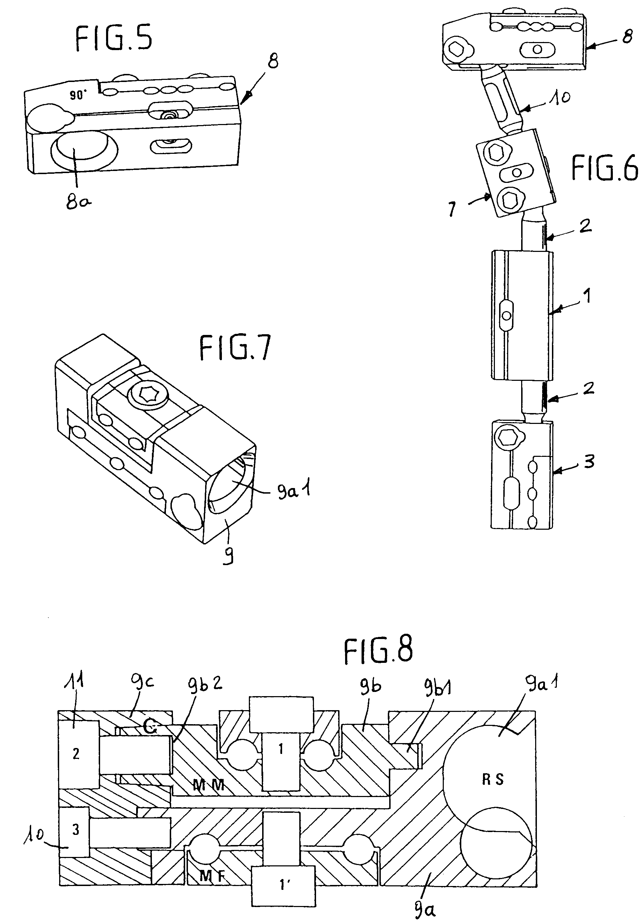

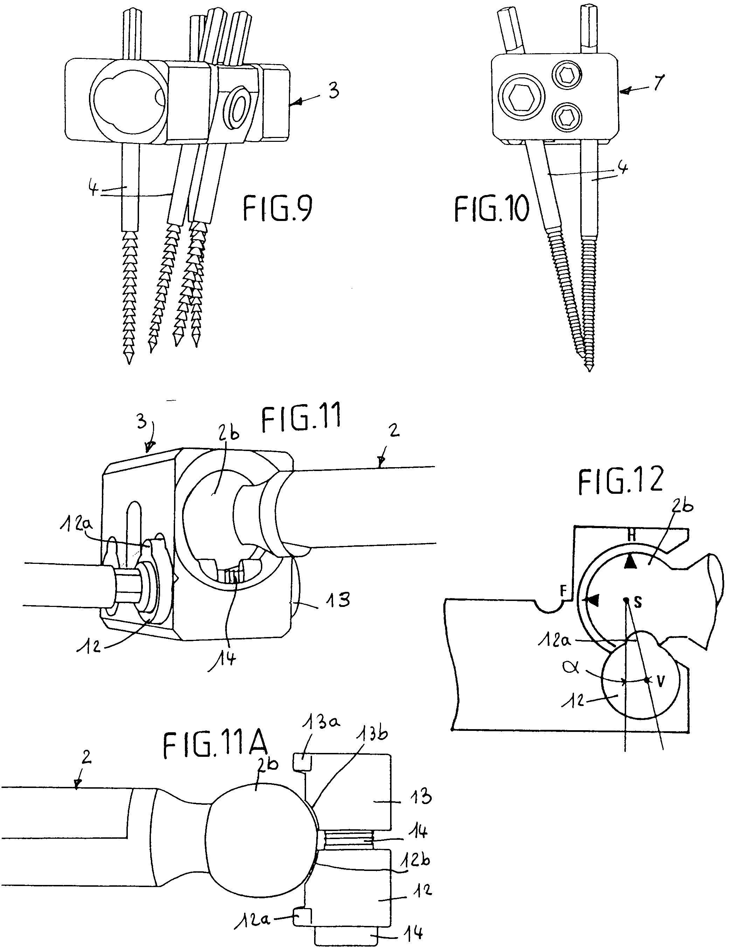

Selon une caractéristique importante de l'invention, la mâchoire à divergence (9) autorise l'implantation de quatre broches d'ancrage pouvant réaliser, deux par deux, un angle aigü dont le sommet doit se situer sur la face d'implantation de l'os (figures 9 et 10). Cette mâchoire (9) présente une partie fixe constituant le corps (9a) et une partie mobile (9b) montée avec capacité de blocage en position angulaire, par rapport au corps (9a). Le corps (9a) et la partie mobile (9b) sont conformés pour recevoir les broches d'ancrage (4) (figure 7).

Dans ce but, la partie mobile (9b) présente, à l'une de ses extrémités, un axe de pivotement (9b1) engagé dans un chambrage interne du corps (9a). L'autre extrémité de la partie mobile (9b) présente une portée tronconique creuse (9b2) engagée dans un chambrage de section complémentaire formé dans un élément rapporté (9c) fixé au corps, au moyen notamment, de vis (10). Un moyen de blocage (11) coopère avec la portée tronconique creuse (9b2) (figure 8). La face transversale du corps (9a) présente une empreinte (9a1) pour l'engagement de la tête sphérique (2a).

Quelle que soit la forme de réalisation des mâchoires, le blocage de la tête sphérique (2a), dans l'empreinte correspondante, s'effectue en combinaison avec des moyens aptes à assurer, dans un premier temps, l'accouplement avec capacité d'orientation angulaire et, dans un deuxième temps, le blocage dans la position angulaire désirée.

Dans ce but, les moyens de blocage sont constitués par deux douilles (12) et (13) engagées chacune dans un trou débouchant dans l'empreinte sphérique correspondante. Chaque douille présente des moyens d'indexation angulaire, sous forme, par exemple, d'un doigt (12a) (13a) coopérant avec une échancrure formée à partir du trou de logement de chacune desdites douilles (figure 11). Les deux douilles (12) et (13) sont assujetties à une vis (14) pour être rapprochées coaxialement en vue d'exercer une force de pression sur la tête sphérique (2b), en combinaison avec des empreintes profilées (12b) (13b) formées aux extrémités des douilles (12) et (13) (figure 11a).

Ces dispositions permettent un montage et démontage, à tout moment d'une intervention, y compris d'une manière pas à pas et y compris sur un fixateur déjà monté, sans en changer d'autres composants.

Les deux douilles (12) et (13), compte-tenu de l'empreinte profilée (12b) (13b) tendent à chasser la tête sphérique (2b). L'axe de la vis de serrage (14) recevant les douilles (12) et (13) est décalé par rapport à la tête sphérique pour obtenir des forces de pression régulièrement réparties dans l'empreinte recevant ladite tête (figure 12).

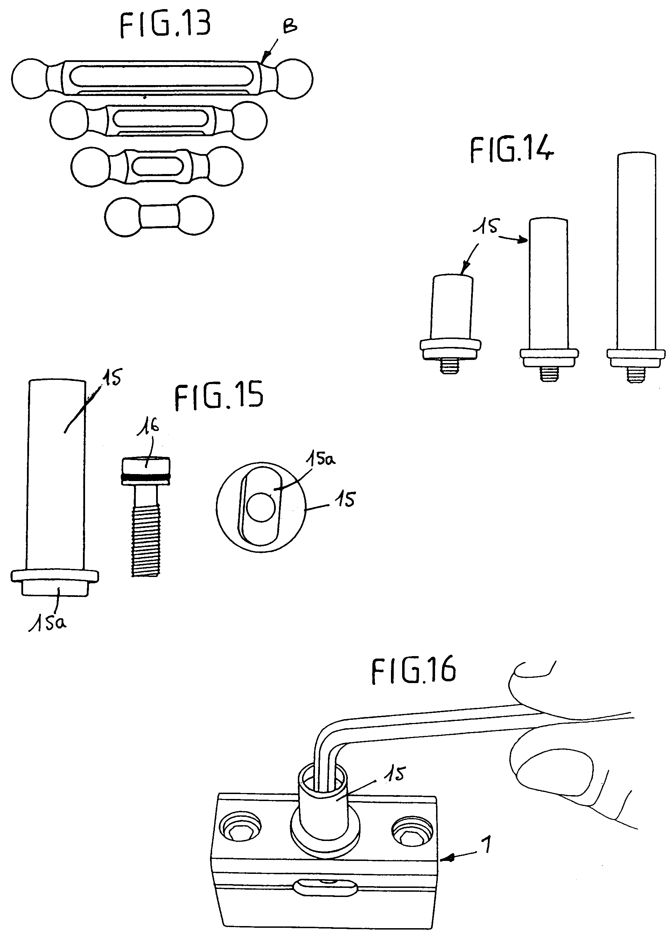

A noter que le corps (1) et les différents types de mâchoires tels que décrits (3) (7) (8) (9), peuvent être accouplés au moyen de barres de mobilisation (B), équipées à chaque extrémité, d'une tête sphérique (figure 13).

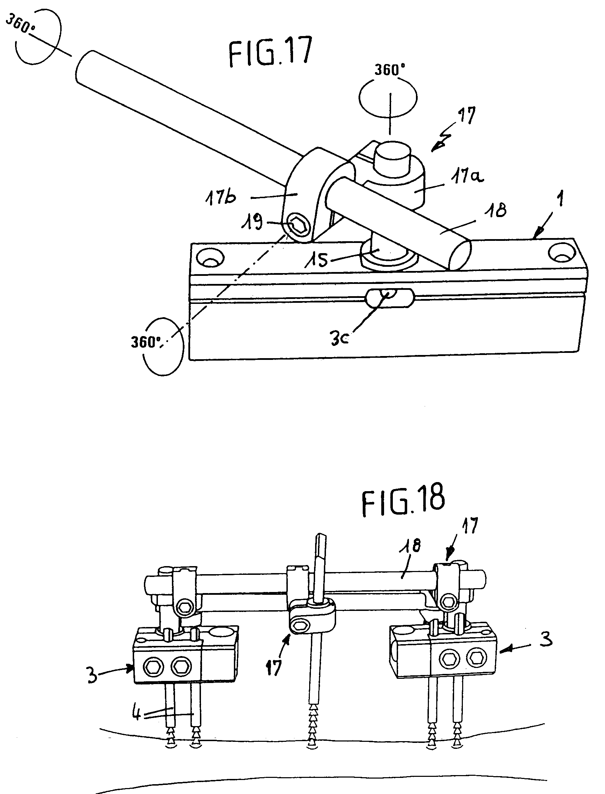

De même, chaque mâchoire (3) (7) (8) (9) présente une empreinte oblongue (3c) destinée à recevoir une portée complémentaire (15a) d'un élément support (15). Cet élément support se présente sous forme d'un corps cylindrique creux, en étant fixé dans ladite empreinte, au moyen d'une vis rapportée (16) (figures 15 et 16). La hauteur du corps cylindrique (15) est variable, notamment cette hauteur présente trois tailles différentes. Le corps cylindrique (15) est destiné à recevoir des colliers de serrage (17).

Chaque collier est en deux parties (17a) (17b), l'une étant engagée sur le corps (15), tandis que l'autre reçoit une barre d'accouplement (18) (figure 17). Une vis de serrage (19) assure le blocage en position du collier (17) qui peut être orienté selon une mobilité de 360° dans tous les plans de l'espace. En combinaison avec un système de cônes auto-bloquants mâle et femelle, la vis (19) assure la liaison du collier sur le corps (15) et de la barre (18) sur ledit collier. Les barres (18) peuvent, de manière connue, assurer la rigidification entre par exemple, deux mâchoires (3), en combinaison avec un support de broches simples (figure 18). Les barres peuvent être exécutées sous forme d'un tube creux en titane anodisé, en matériau composite, en carbone époxy... Les barres présentent plusieurs longueurs.

A noter que dans le cas où toutes les broches d'ancrage pour une mâchoire, ne sont pas nécessaires, le logement libre est obturé par un élément neutre qui a pour but d'assurer l'équilibrage des forces de pression exercées par les mâchoires.

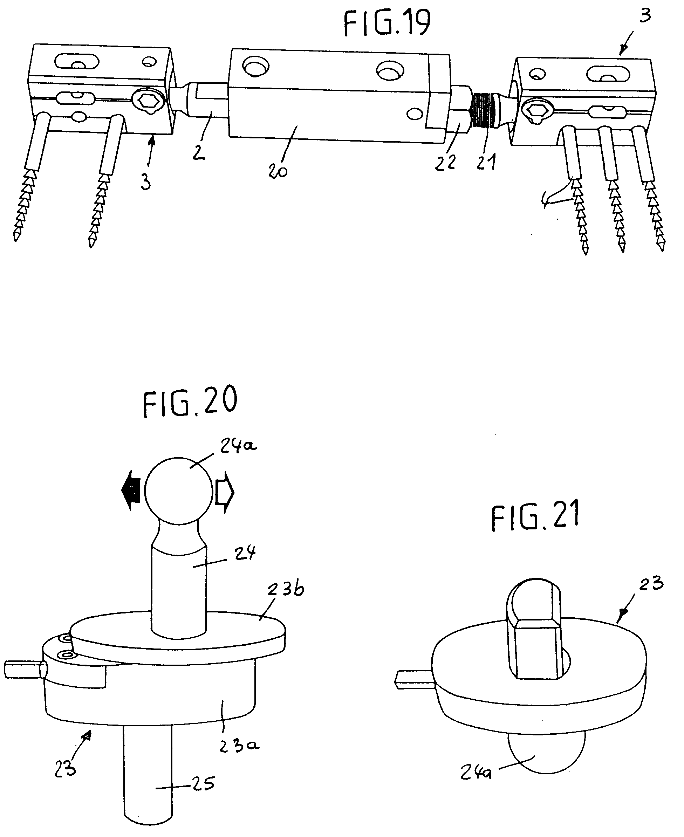

Suivant une autre caractéristique, les mâchoires peuvent être réunies par un corps d'allongement (20) (figure 19). Ce corps présente, à l'une de ses extrémités, au moins un trou taraudé recevant la tige d'un piston (21) qui est filetée pour être déplacée en translation, en combinaison avec un écrou (22). L'autre extrémité du corps (20) présente un trou pour l'engagement d'une tige (2) dont le blocage en translation s'effectue de la même façon qu'avec le corps (1).

Pour faciliter la réduction d'une fracture, il est prévu d'intercaler entre un corps et une mâchoire, un module d'alignement (23) (figures 20 et 21). Ce module comprend deux parties oblongues (23a) (23b) accouplées entre elles, avec capacité de désaxation latérale contrôlée dans deux plans perpendiculaires. L'une des parties (23a) reçoit une tige (24) équipée en bout d'une tête sphérique (24a) destinée à être accouplée à une mâchoire, tandis que l'autre partie (23b) reçoit une tige (25) avec méplat, destinée à être rendue solidaire du corps.

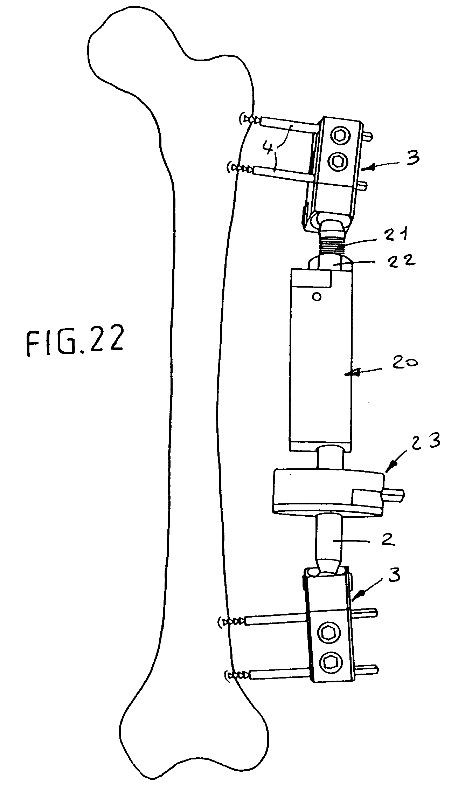

La figure 22 montre un exemple de montage avec un module d'allongement.

Compte-tenu des différents éléments et modules composants le fixateur selon l'invention, il peut être envisagé une pluralité de montages pour traiter une pluralité de cas pathologiques, d'une manière modulaire et évolutive et avec une grande simplicité d'utilisation.

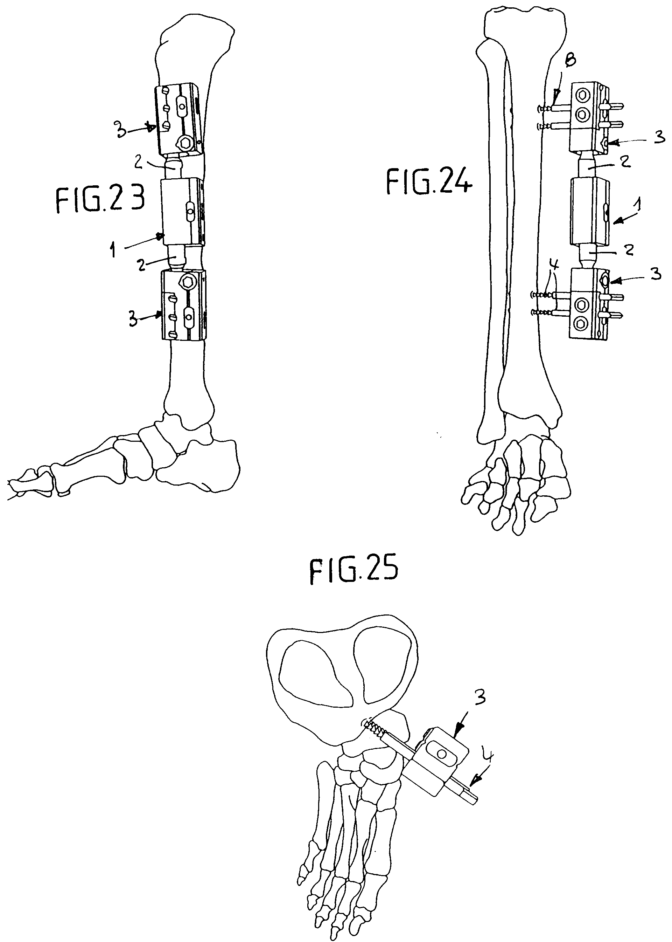

Les figures 23 et suivantes, montrent des exemples nullement limitatif de montages possibles avec les éléments du fixateur selon l'invention.

Les figures 23, 24 et 25 montrent un montage tibial au niveau de la diaphyse, au moyen d'un corps (1) et de deux mâchoires (3).

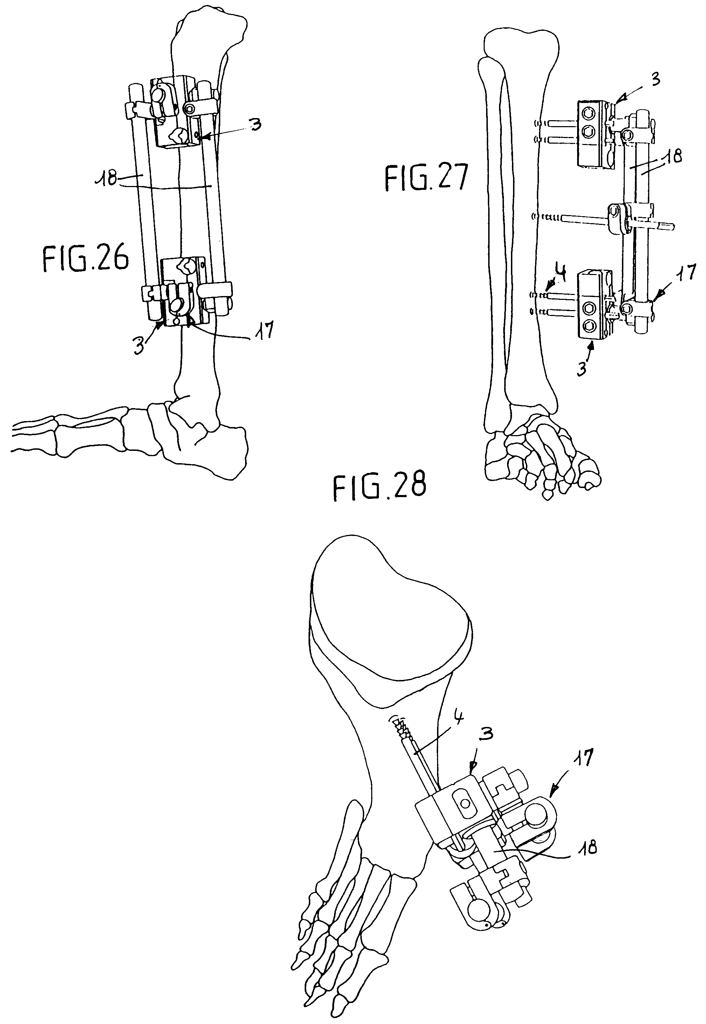

Les figures 26, 27 et 28 montrent un montage diaphysaire au moyen de deux barres (18) reliant deux mâchoires (3).

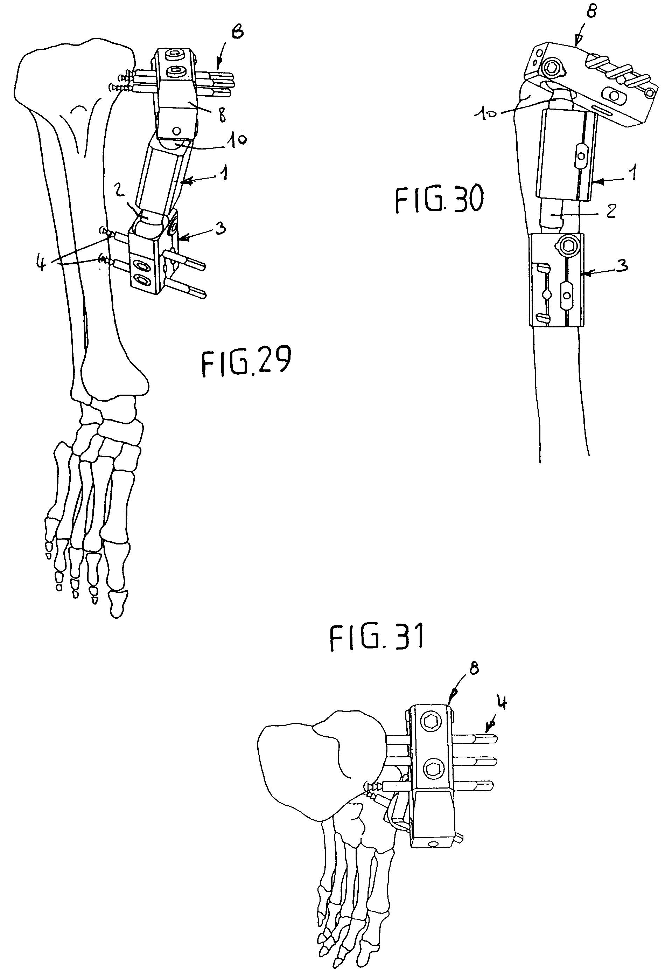

Les figures 29, 30 et 31 montrent un montage au niveau épiphysaire utilisant un corps (1) monté entre une mâchoire standard (3) et une mâchoire à 90° (8).

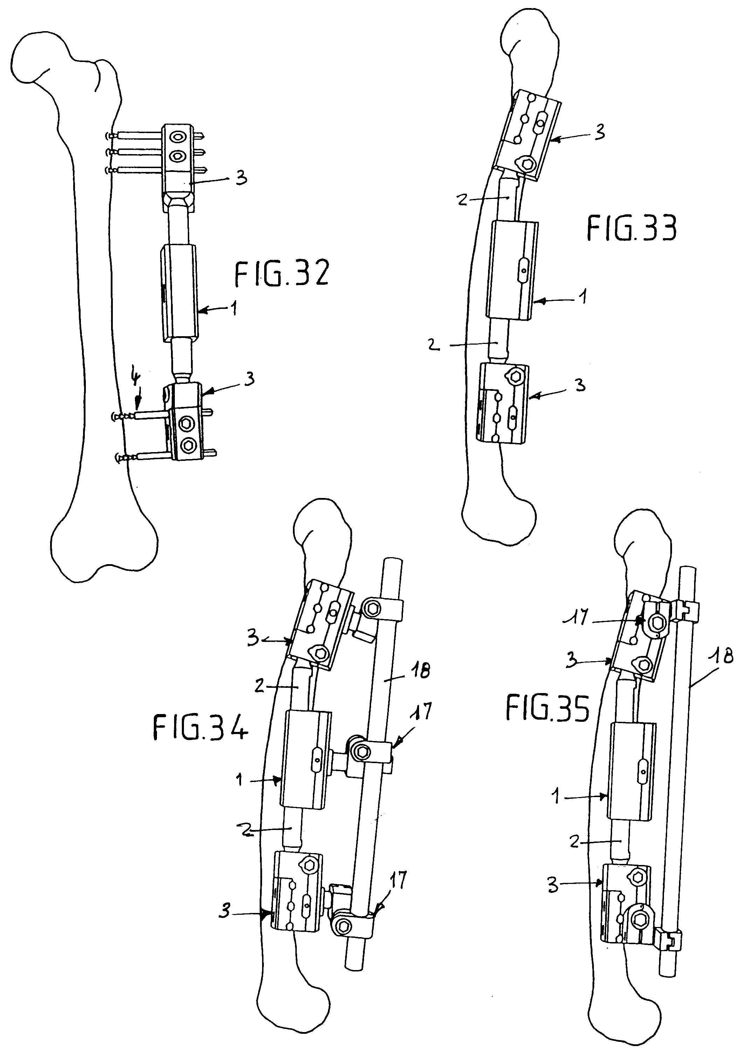

Les figures 32 et 33 montrent un montage diaphysaire mono-latéral mettant en oeuvre un corps (1) monté entre deux mâchoires standards (3), tandis que les figures 34 et 35 montrent un montage diaphysaire utilisant une barre de rigidification (18) et un relais sur le corps.

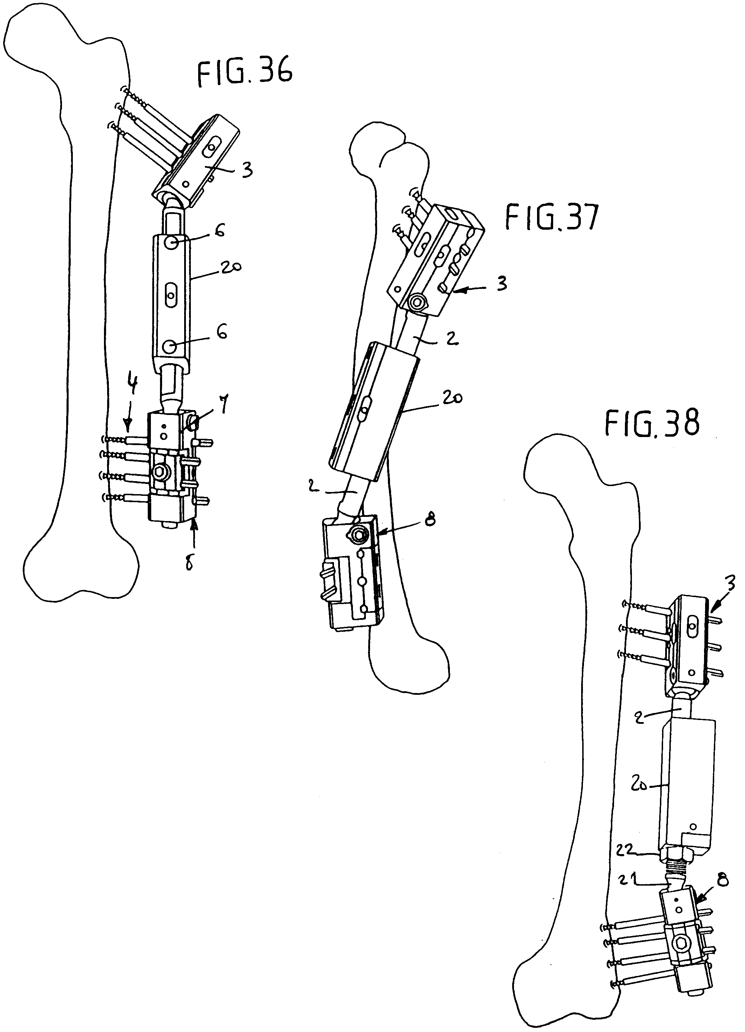

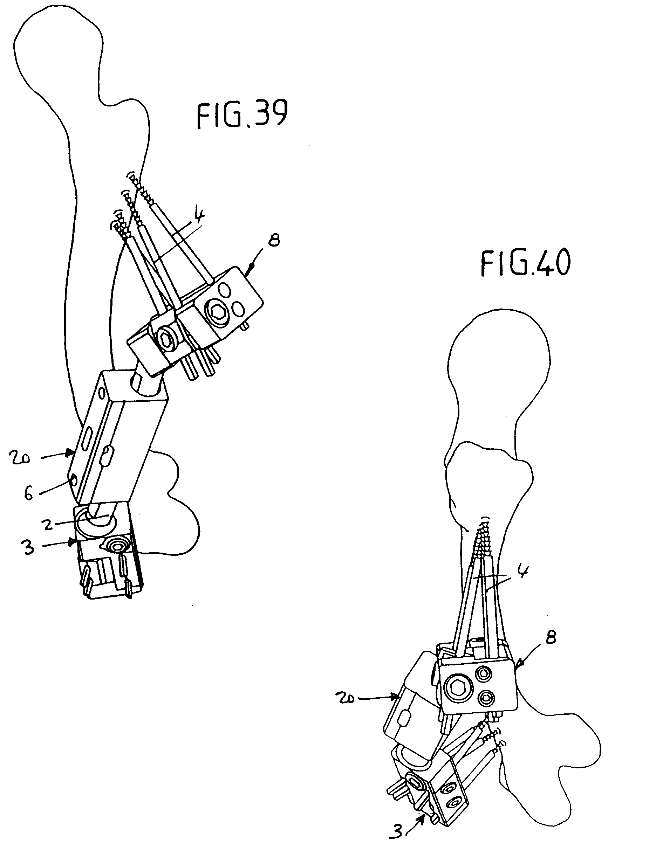

Les figures 36, 37, 38, 39 et 40 montrent un montage épiphysaire mettant en oeuvre un corps d'allongement (20) monté entre une mâchoire standard (3) et une mâchoire divergente (7).

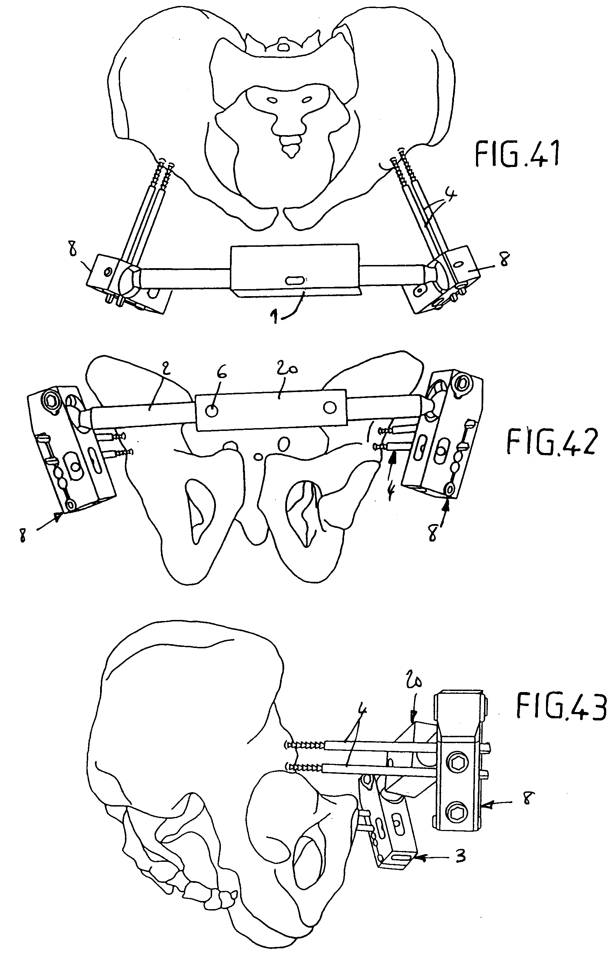

Les figures 41, 42 et 43 montrent un montage standard au niveau du bassin, utilisant un corps (1) accouplé entre deux mâchoires à 90° (8).

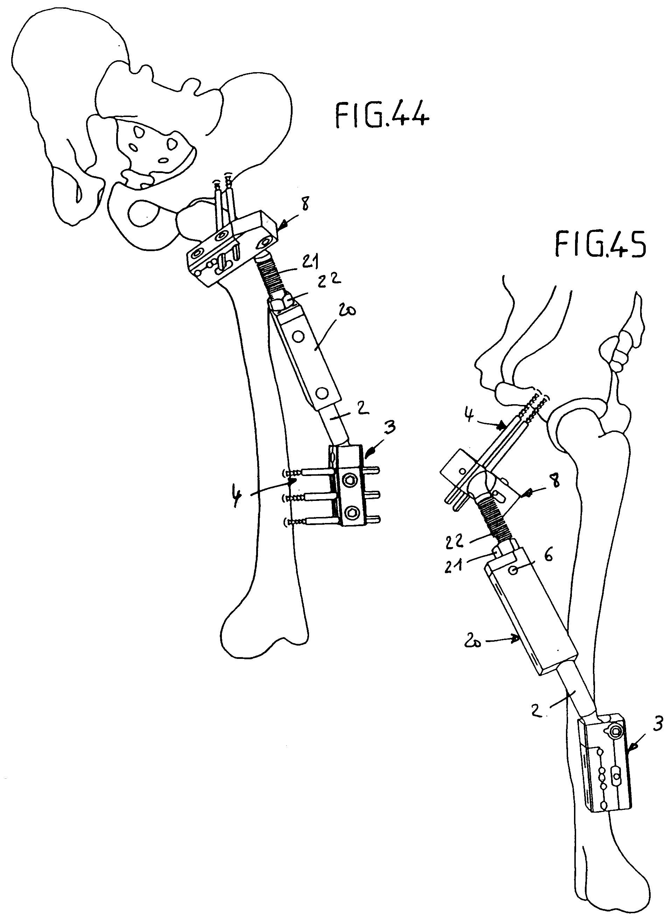

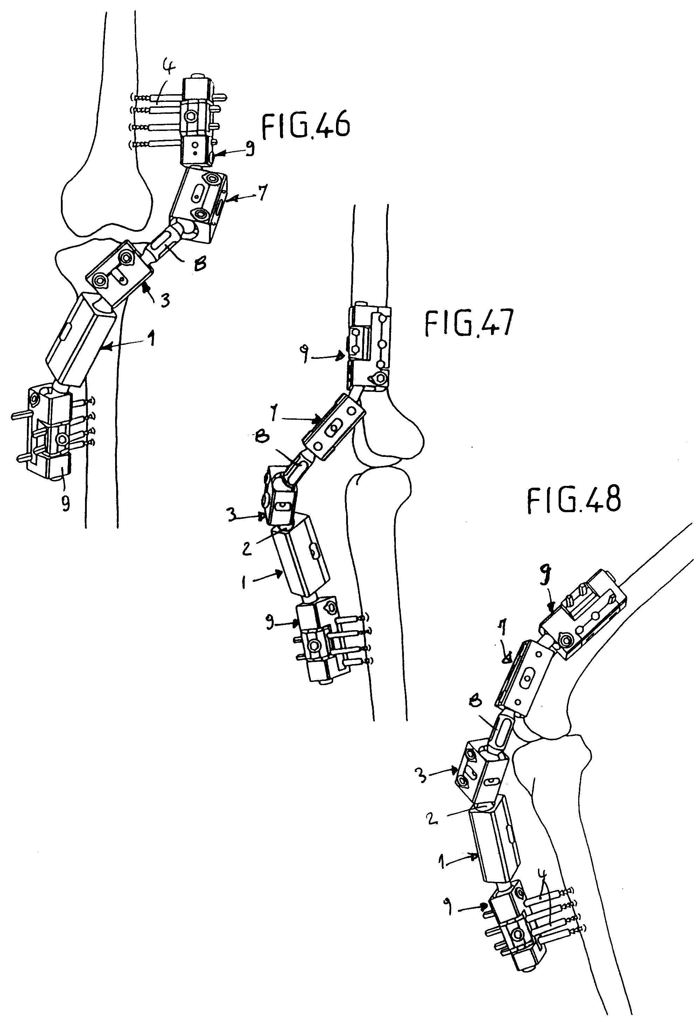

Les figures 44 et 45 montrent l'immobilisation de l'articulation coxo fémorale au moyen d'un corps d'allongement (20) accouplé entre une mâchoire standard (3) et une mâchoire à 90° (8).

Les figures 46, 47 et 48 montrent un montage entre le fémur et le tibia au moyen de deux broches divergentes accouplées au moyen de deux relais et d'un corps d'allongement. Ces dispositions permettent d'atteindre la face interne du tibia et de distracter l'articulation si nécessaire. Les montages obtenus peuvent être effectués pour une flexion du genou allant de 0 à 40°.

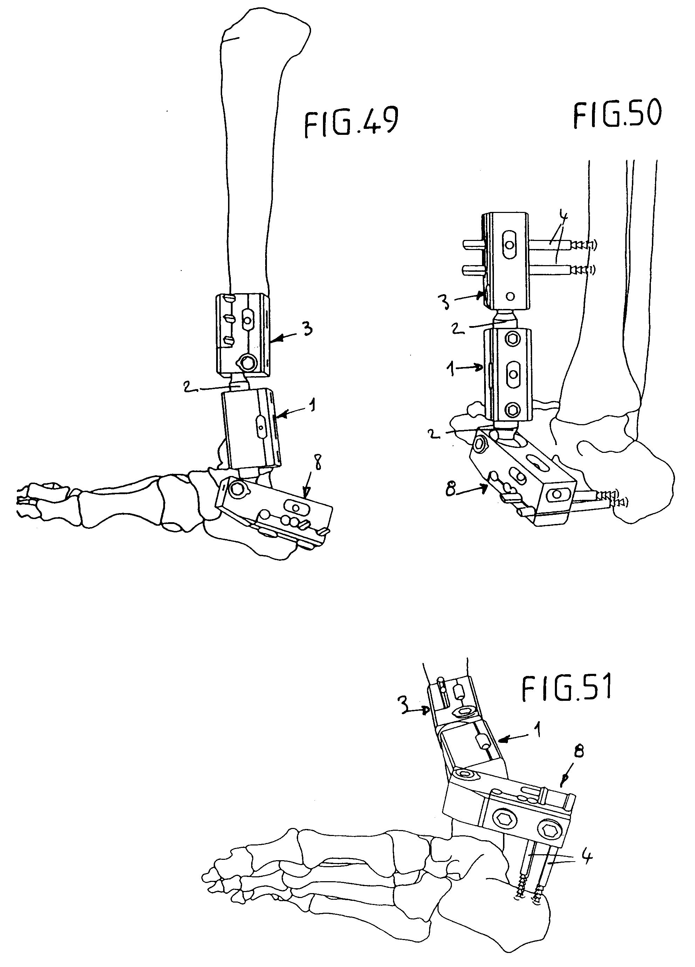

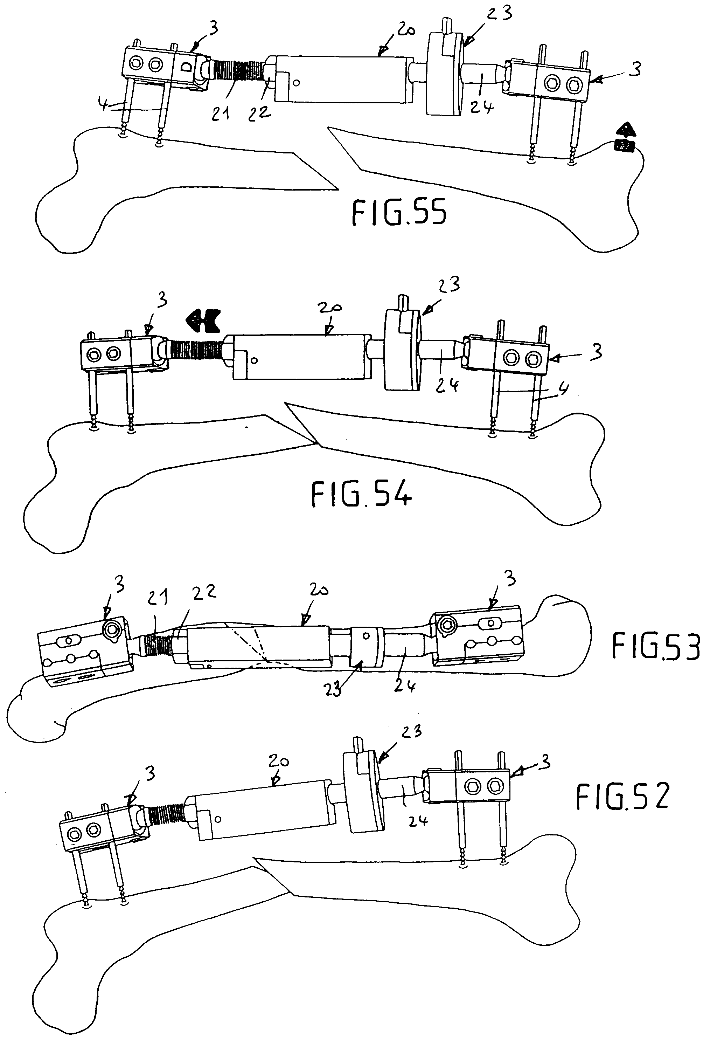

Les figures 49, 50 et 51 montrent l'adaptation du fixateur au niveau de l'articulation de la cheville, au moyen d'une mâchoire standard et d'une mâchoire à 90° (8) reliée à un corps (1). La mâchoire à 90° est disposée en face du centre de la demi-sphère astragalienne.

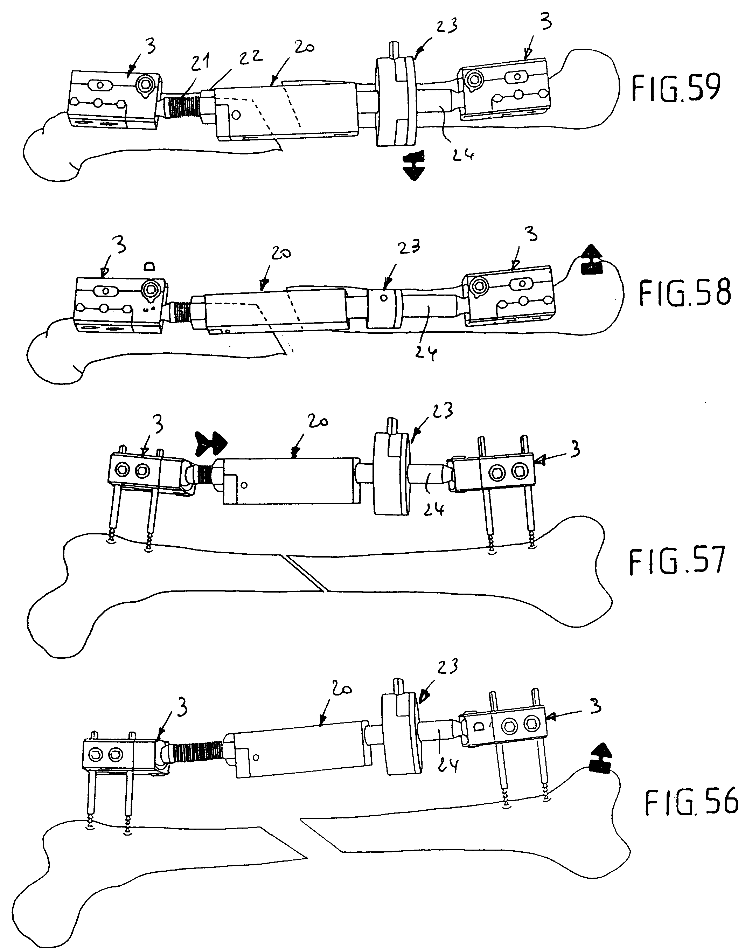

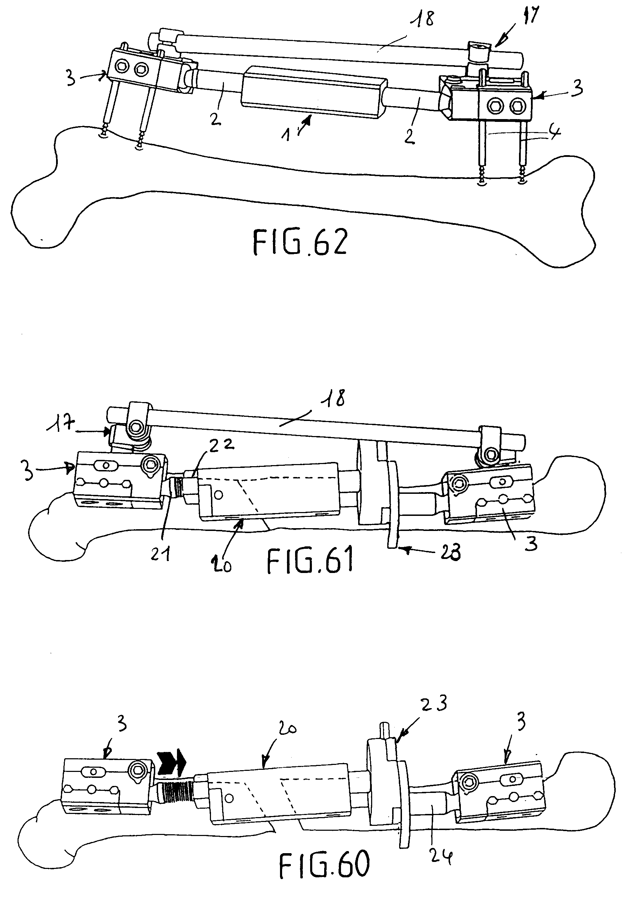

Les figures 52, 53, 54, 55, 56, 57, 58, 59, 60, 61 et 62 montrent des fractures à dominante de flexion utilisant deux mâchoires standards (3) accouplées par un corps d'allongement (20) en combinaison avec un module d'alignement (23). Les mâchoires (3) sont positionnées en sus et sous fracturaire, les broches d'ancrage étant disposées perpendiculairement à l'os (figures 52 et 53). Le corps d'allongement (20) permet de retrouver la longueur (figures 54 et 55). La mâchoire supérieure est ensuite débloquée angulairement pour aligner, au moyen du module (23), le corps du fixateur sur le fragment supérieur, la mâchoire est ensuite bloquée en position. On procède de la même façon pour la mâchoire inférieure. Le corps d'allongement (20) est racourci pour retrouver la bonne longueur. Les mâchoires supérieure et inférieure sont débloquées et le module d'alignement tourné de 90° pour réduire le décalage. Si le module est en bout de course, on utilise une barre relais et on enlève ensuite ledit module et le corps d'allongement (figure 61). Une fois la réduction obtenue, la barre de rigidification (18) permet d'enlever l'ensemble du corps d'allongement (20) et du module et de les remplacer par un corps (1) accouplé aux mâchoires (3) par les tiges pistons (2) (figure 62).

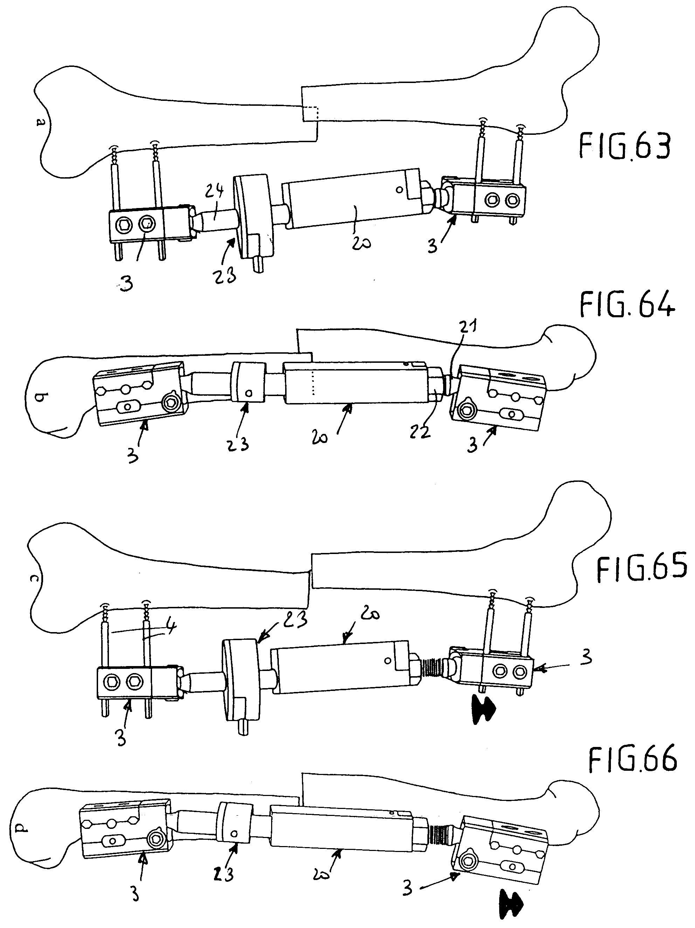

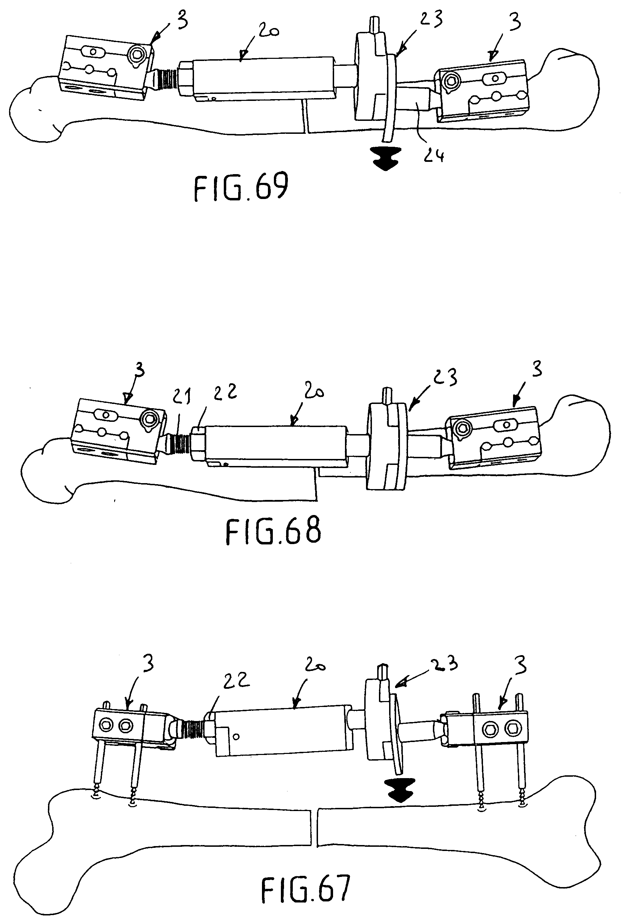

Les figures 63, 64, 65, 66, 67, 68 et 69 montrent une réduction de fracture à dominante de translation au moyen de deux mâchoires standards en combinaison avec un corps d'allongement (20) et un module d'alignement (23).

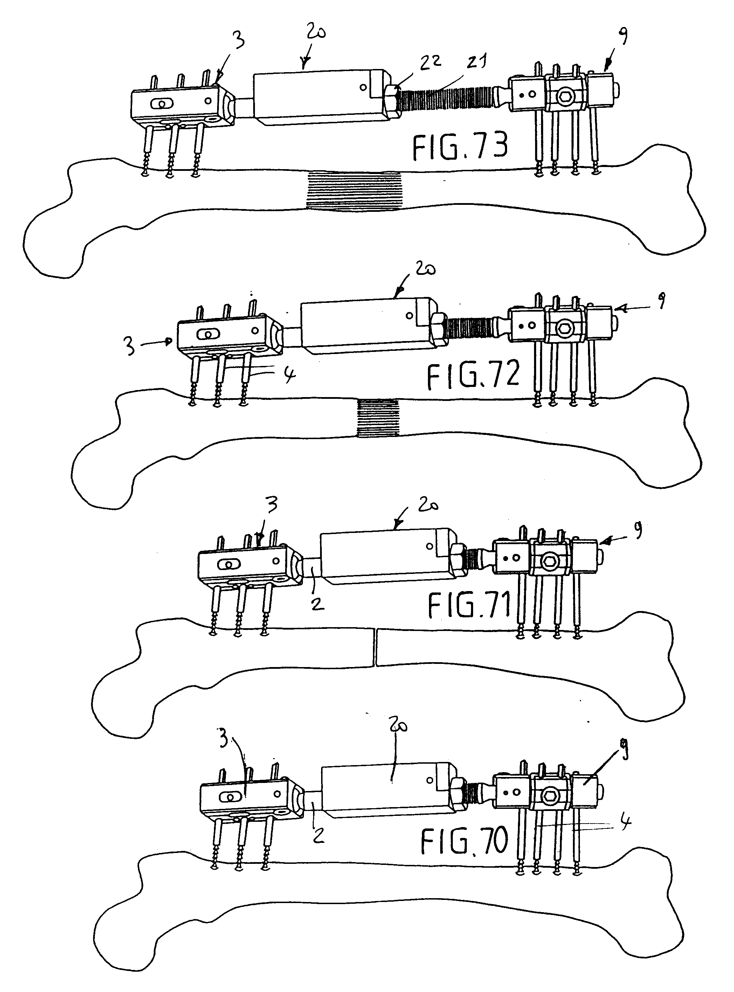

Les figures 70, 71, 72 et 73 montrent un montage du fixateur au moyen d'une mâchoire standard (3) et d'une mâchoire divergente (9) réunies par un corps d'allongement (20) pour obtenir un allongement diaphysaire fémoral.

Les avantages ressortent bien de la description, en particulier on souligne et on rappelle:
- le caractère modulable du fixateur permettant de passer d'un montage à un autre sans anesthésie, sans modification des forces exprimées au niveau du foyer de fracture et sans modification de la morphologie de ce foyer,
- la possibilité d'enlever un ou plusieurs éléments du fixateur de sorte que ce dernier peut évoluer en fonction de l'évolution de la fracture,
- la simplicité d'utilisation,
- la possibilité d'obtenir l'alignement d'une fracture grâce à un ancillaire spécifique,
- la possibilité d'obtenir le mouvement d'une articulation mise en distraction,
- le passage d'un système à un autre, au moyen des éléments rapportés et aptes à être fixés sur les mâchoires.

## Revendications

**-1-** Fixateur externe modulaire évolutif pour immobilisation d'un foyer de fracture, comprenant des éléments faisant office de mâchoires (3) et recevant des broches d'ancrage (4) pour être fixées dans une position angulaire déterminée de part et d'autre du foyer de fracture, lesdites mâchoires (3) étant aptes à recevoir, après fixation, au moins un corps (1) avec capacité d'orientation angulaire contrôlée et de blocage dans cette position au moyen de tiges d'accouplement (2), les tiges d'accouplement (2), entre le corps et les mâchoires, étant constituées par une portée cylindrique (2a) faisant office de piston et destinée à être engagée avec capacité de coulissement et de blocage en translation dans un agencement complémentaire du corps (1), l'une des extrémités de ladite portée présentant une tête sphérique (2b) apte à coopérer dans un logement complémentaire formé dans les mâchoires avec capacité de blocage angulaire, caractérisé en ce qu'il comprend :
- des mâchoires standards (3) conformées pour être fixées coaxialement selon différentes positions angulaires avec le corps (1) et/ou d'autres mâchoires,
- des mâchoires intermédiaires (7) conformées pour être fixées coaxialement selon différentes positions angulaires avec d'autres mâchoires ou une mâchoire et un corps,
- des mâchoires (8) conformées pour être fixées avec le corps et/ou d'autres mâchoires dans un plan orthogonal,
- des mâchoires (9) dont une partie est orientable pour le positionnement angulaire des broches d'ancrage,
- un corps d'allongement (20) apte à être monté entre deux mâchoires,
- un module d'alignement (23),

**-2-** Fixateur selon la revendication 1, caractérisé en ce que les tiges (2) des pistons sont de différentes longueurs et présentent une portée méplate (2a) coopérant avec une vis de blocage (6) du corps (1), les extrémités débordantes desdites tiges qui présentent la tête sphérique (2b), coopérant avec des empreintes en creux complémentaires des mâchoires, sont bloquées en position angulaire par des moyens du type clavette.

**-3-** Fixateur selon la revendication 2, caractérisé en ce que les moyens de blocage des têtes sphériques (2b) sont constitués par deux douilles (12) (13) engagées chacune dans un trou débouchant dans l'empreinte recevant la tête sphérique en y étant indexées angulairement, lesdites douilles étant assujetties à une vis (14) pour être rapprochées coaxialement en vue d'exercer une force de pression sur ladite tête, en combinaison avec des empreintes (12b) (13b) profilées formées aux extrémités desdites douilles.

**-4-** Fixateur selon la revendication 1, caractérisé en ce que les mâchoires (8), conformées pour être fixées avec le corps et/ou d'autres mâchoires, dans un plan orthogonal, présentent au moins une empreinte (8a) formée dans l'épaisseur de l'une de ses faces latérales, pour l'engagement et la fixation de la tête sphérique (2b) d'une tige piston de liaison.

**-5-** Fixateur selon la revendication 1, caractérisé en ce que la mâchoire orientable (9) présente une partie fixe (9a) pour l'engagement de broches d'ancrage et une partie mobile (9b) avec capacité de blocage en position angulaire, ladite partie mobile recevant également des broches d'ancrage.

**-6-** Fixateur selon la revendication 5, caractérisé en ce que la partie mobile (9b) présente, à une de ses extrémités, un axe de pivotement (9b1) engagé dans une portée interne de la partie fixe (9a) et, à l'autre extrémité, une portée tronconique creuse engagée dans un chambrage formé dans un élément rapporté (11) du corps, un moyen de blocage coopérant avec l'alésage de la portée tronconique.

**-7-** Fixateur selon la revendication 1, caractérisé en ce que le corps d'allongement (20) présente, à l'une de ses extrémité au moins, un trou taraudé recevant la tige (21) d'un piston qui est filetée pour être déplacée en translation par un écrou (22) bloqué en translation au niveau de ladite extrémité.

**-8-** Fixateur selon la revendication 1, caractérisé en ce que le module d'alignement (23) comprend deux parties oblongues (23a) (23b) accouplées entre elles avec capacité de désaxation latérale l'une des parties recevant la tige (2) d'un piston coopérant avec un corps, l'autre partie recevant une tête sphérique (24a) coopérant avec une mâchoire.

**-9-** Fixateur selon la revendication 1, caractérisé en ce que le corps (1) et/ou la mâchoire présente une empreinte oblongue destinée à recevoir une portée complémentaire d'un élément support (15) présentant des agencements de fixation au niveau de ladite empreinte, ledit élément support étant apte à recevoir les parties d'un collier de serrage (17) recevant des barres de rigidification (18) entre plusieurs corps et/ou mâchoire.
